# EUROPEAN PATENT APPLICATION

(11) **EP 4 258 181 A1**
(43) Date of publication of application: **11.10.2023**
(21) Application number: 22167201.7
(22) Date of filing: 07.04.2022
(51) Int. Cl.: G06N 3/10, G06N 3/04

(54) **METHODS AND SYSTEMS FOR ADAPTING AN ARTIFICIAL NEURAL NETWORK GRAPH**

(71) Applicant: Aptiv Technologies Limited, St. Michael (BB)
(72) Inventor: Bücs, Róbert Lajos, 42275 Wuppertal (DE); Aguilar, Miguel Angel, 40235 Düsseldorf (DE)
(74) Representative: Manitz Finsterwald Patent- und Rechtsanwaltspartnerschaft mbB

(57) **Abstract**

A computer implemented method for adapting an artificial neural network graph, the method comprising: acquiring an input artificial neural network graph; carrying out a global manipulation step comprising a first set of manipulations configured to adapt the input artificial neural network graph based on at least one user-defined criterion to generate a first intermediate artificial neural network graph; dissecting the first intermediate artificial neural network graph to generate a plurality of second intermediate artificial neural network graphs based on a specification file; and carrying out a respective local manipulation step for each of the plurality of second intermediate artificial neural network graphs, wherein each local manipulation step comprises a corresponding second set of manipulations configured to adapt a corresponding second intermediate artificial neural network graph based on at least one corresponding user-defined criterion and generate a corresponding manipulated second intermediate artificial neural network graphs.

## Description

### FIELD

The present disclosure relates to methods and systems for adapting an artificial neural network graph.

### BACKGROUND

Various processing methods may be represented by artificial neural network graphs. Not all artificial neural network graphs may be deployed directly, and as such, it may be cumbersome to handle the artificial neural network graphs.

Accordingly, there is a need to provide methods for manipulating artificial neural network graphs.

The present disclosure addresses, inter alia, the challenge of deployment of machine learning applications. "Deploying" may refer to embodying an artificial neural network, for example represented by a computational graph, in a system for application (in contrast to embodying the computational graph for analysis purposes or training purposes). In this context, 'graph surgery' refers to a process which includes dissection (of the artificial neural network graph, for example dissection into several smaller graphs), and graph manipulations before and after the dissection.

Whetton et al. (B. Whetton, "Keras Surgeon," [Online]. Available: https://github.com/BenWhetton/keras-surgeon) present a software library for model manipulation directly in a particular training framework. However, the implemented graph-level modifications of the tool mostly revolve around pruning, i.e., a model compression technique aiming at removing redundant parts of a network. Thus, its features related to graph surgery include only deleting neurons and channels, as well as deleting, inserting and replacing complete layers. Moreover, the library is bound to a particular training framework and does not semantically separate thus training from deployment. Furthermore, Whetton et al. do not consider graph manipulation mechanisms, as provided by various embodiments.

GraphSurgeon (TensorRT, "GraphSurgeon," Nvidia, [Online]. Available: https://docs.nvidia.com/deeplearning/tensorrt/api/python_api/graphsurgeon/graphs urgeon.html) is another software library integrated into NVIDIA's TensorRT deployment framework. It works on the serialized graph representation of ANN (artificial neural network) models, which enables the previously suggested semantic separation between training and deployment. GraphSurgeron provides an intermediate graph wrapper which allows more generic ways of using basic graph manipulations, e.g., creating / modifying / removing nodes and layers, as well as replacing even complete subgraphs. However, GraphSurgeon does not consider any higher-level nor subgraph-specific manipulation mechanisms, as provided by various embodiments.

### SUMMARY

The present disclosure provides a computer implemented method, a computer system and a non-transitory computer readable medium according to the independent claims. Embodiments are given in the subclaims, the description and the drawings.

In one aspect, the present disclosure is directed at a computer implemented method for adapting an artificial neural network graph, the method comprising: acquiring an input artificial neural network graph; carrying out a global manipulation step comprising a first set of manipulations configured to adapt the input artificial neural network graph based on at least one user-defined criterion and generate a first intermediate artificial neural network graph; dissecting the first intermediate artificial neural network graph to generate a plurality of second intermediate artificial neural network graphs based on a specification file; and carrying out a respective local manipulation step for each of the plurality of second intermediate artificial neural network graphs, wherein each local manipulation step comprises a corresponding second set of manipulations configured to adapt a corresponding second intermediate artificial neural network graph based on at least one corresponding user-defined criterion and generate a corresponding manipulated second intermediate artificial neural network graph.

With the method according to various embodiments, a global manipulation may be carried out (for example to an overall artificial neural network graph), followed by dissection (which may for example "cut" various parts of the overall artificial neural network graph), and then local manipulations to the various cut out parts. This may provide a flexible and efficient way of enhancing deployability of artificial neural network graphs.

According to various embodiments, the method further comprises converting the at least one manipulated second intermediate artificial neural network graph to an on-board format, that is directly executable on the system on which the input artificial neural network graph is to be deployed (e.g. target hardware / embedded system). The on-board format may depend on the target hardware or embedded system.

According to various embodiments, the first set of manipulations and/or the second set of manipulations are provided in a configuration file, preferably a textual configuration file. This may allow a user to tailor the method to the specific requirements of the user and may allow easy configurability.

According to various embodiments, dissecting comprises dissecting the first intermediate artificial neural network graph at pre-defined points of intersection.

According to various embodiments, the points of intersection are provided in a configuration file, preferably a textual configuration file. This may allow a user to tailor the method to the specific requirements of the user and may allow easy configurability.

According to various embodiments, the input artificial neural network graph is provided in an off-board format. The off-board format may not directly be used on the system on which the input artificial neural network graph is to be deployed, but may be used for manipulating the input artificial neural network graph.

According to various embodiments, carrying out the respective local manipulation step comprises carrying out individual manipulations to at least two second intermediate artificial neural network graphs. The individual manipulations may be specific to each of the at least two second intermediate artificial neural network graphs. In other words, the manipulation applied to a first one of the at least two second intermediate artificial neural network graphs may be different from the manipulation applied to a second one of the at least two second intermediate artificial neural network graphs.

According to various embodiments, each of the input artificial neural network graph, the first intermediate artificial neural network graph, the plurality of second intermediate artificial neural network graphs, and the at least one manipulated second intermediate artificial neural network graph comprises a respective plurality of nodes representing mathematical operations and a respective plurality of edges representing tensors.

According to various embodiments, the method further comprises visualizing at least one graph selected from a list of graphs consisting of: the input artificial neural network graph, the first intermediate artificial neural network graph, the plurality of second intermediate artificial neural network graphs, and the at least one manipulated second intermediate artificial neural network graph. The visualization may provide visual feedback to the user, and may thus enhance the user experience.

According to various embodiments, the at least one manipulated artificial neural network graph is to be deployed on a resource-constrained embedded system.

According to various embodiments, the embedded system is a mobile computing device, a mobile phone, a tablet computing device, an automotive compute platform, or an edge device.

In another aspect, the present disclosure is directed at a computer system, said computer system comprising a plurality of computer hardware components configured to carry out several or all steps of the computer implemented method described herein.

Each of the computer system and the embedded system may comprise a plurality of computer hardware components, for example a processor, for example processing unit or processing network, at least one memory, for example memory unit or memory network, and at least one non-transitory data storage. It will be understood that further computer hardware components may be provided and used for carrying out steps of the computer implemented method in the computer system. The non-transitory data storage and/or the memory unit may comprise a computer program for instructing the computer to perform several or all steps or aspects of the computer implemented method described herein, for example using the processing unit and the at least one memory unit.

In another aspect, the present disclosure is directed at a non-transitory computer readable medium comprising instructions for carrying out several or all steps or aspects of the computer implemented method described herein. The computer readable medium may be configured as: an optical medium, such as a compact disc (CD) or a digital versatile disk (DVD); a magnetic medium, such as a hard disk drive (HDD); a solid state drive (SSD); a read only memory (ROM), such as a flash memory; or the like. Furthermore, the computer readable medium may be configured as a data storage that is accessible via a data connection, such as an internet connection. The computer readable medium may, for example, be an online data repository or a cloud storage.

The present disclosure is also directed at a computer program for instructing a computer to perform several or all steps or aspects of the computer implemented method described herein.

The methods and devices according to various embodiments may provide a graph surgery methodology, for example for optimizing ANN (artificial neural networks). Surgery may be performed once the model is serialized in its graph representation. This separation may emphasize the isolation between the modelling and training processes from the deployment flow, latter which frequently requires specific graph alterations to ensure compatibility with the framework in which the graph is to be deployed.

Various embodiments may provide a semantic separation between the training and deployment flow by applying manipulations on ML (machine learning) graph formats. This representation lies exactly between training and deployment, thus being conceptually independent from both worlds.

Various embodiments may separate the concept of pre- and post-surgery manipulations, on top of generic graph manipulations, which notions are imminent in practical graph surgery flows.

Various embodiments may use a simple textual configuration file to specify details for graph surgery, to enable the user for providing particular manipulations to the graph, and for providing implementation-specific output configurations for the final graph conversion.

Various embodiments may provide a software interface to perform pre-surgery global graph modifications, as well as post-surgery local manipulations on particular subnets only. This interface, firstly, lays down the structure which custom manipulation passes need to follow to be executed by our tool, but also specifies a set of utility functions which can be used in the custom code. This mechanism allows the user to plug in user-specific code without exposal, which may be beneficial in a production environment.

Various embodiments may provide both high-level and in-depth visual feedback to the user which may allow to inspect, understand and verify the results of graph surgery. The visual feedback may be beneficial for debugging possible issues with the deployment tool, since input graphs can be dissected on a node-level granularity, allowing a detailed divide-and-conquer approach.

Various embodiments may provide dissecting networks to isolate subgraphs which are fully deployable from ones whose mathematical operations are not supported by the target deployment tool; and/or dissecting networks to enable heterogeneous execution of the subgraphs. On the one hand, this may allow to distribute subnets to the best suitable core of a heterogeneous hardware architecture, i.e., which executes the subnet fastest. On the other hand, it allows simultaneous execution of subgraphs, if these do not have inter-dependencies.

### DRAWINGS

Exemplary embodiments and functions of the present disclosure are described herein in conjunction with the following drawings, showing schematically:
- Fig. 1: an illustration of a high-level block diagram of a system according to various embodiments;
- Fig. 2: an illustration of a meta-implementation of a graph surgery specification file and the corresponding subnetwork outputs according to various embodiments;
- Fig. 3: an illustration of a high-level graph surgery visualization and a detailed graph surgery visualization according to various embodiments;
- Fig. 4: a flow diagram illustrating a method for adapting an artificial neural network graph according to various embodiments;
- Fig. 5: an enhancement system according to various embodiments; and
- Fig. 6: a computer system with a plurality of computer hardware components configured to carry out steps of a computer implemented method for adapting an artificial neural network graph according to various embodiments.

### DETAILED DESCRIPTION

Machine learning (ML) has become widely adopted in a variety of fields nowadays, including computer vision, automotive, financial, medical, and numerous others, since such applications may significantly outperform classic methods on the very same task. ML models may be realized as so-called artificial neural networks (ANNs): structures that can be trained to effectively learn certain tasks. The superiority of ANNs has resulted in a paradigm shift for deploying such networks on resource-constrained embedded systems, in addition to general-purpose computers.

The embedded transition, however, poses major deployment challenges since ANNs are computation-intensive algorithms which need now to execute on constrained hardware. This problem may persist despite the evolution of such devices over time; integrating powerful, heterogeneous, multi- and many-core CPUs (central processing units), ANN accelerators, as well as a significant amount of memory. To address this challenge and exploit hardware features in the best way possible, vendors (as well as third-party suppliers) provide deployment tool chains for their devices. Such frameworks implement mechanisms to tailor the networks for highly efficient execution on the target hardware, given the ANNs in a certain standard-compliant exchange format. These formats were established to effectively store and serialize structured data into binary or textual form, or even as source code. In such formats, ANNs are typically represented as static artificial neural network graphs with mathematical operations as nodes and tensors as edges, i.e., structures that enable data flow between nodes. Such graph formats are independent of the model formats used by ANN training frameworks. This distinction separates the modeling and training processes from the hardware-specific deployment flow.

However, deployment tools may support only a limited subset of available mathematical operations for ANNs, posing yet another deployability challenge. Moreover, certain tools may leave the decision to the user on which core/ accelerator of the heterogeneous hardware to execute the ANN, posing an additional performance exploration challenge.

Such challenges may be addressed by using graph surgery, i.e., the capability to split ANNs into subnetworks. On the one hand, this technique allows dissecting ANNs into chunks which are fully supported by the given deployment tool and other chunks which need to be re-created and deployed perhaps manually. On the other hand, graph surgery may enable the possibility to execute the created subnetworks in a distributed manner, if this would not be automated by the deployment tool chain.

Graph surgery may take place in the ANN training tool. However, according to various embodiments, this action may be performed once the model is serialized in its graph representation. This separation may emphasize the isolation between the modeling and training processes from the deployment flow, latter which frequently requires specific graph alterations to ensure compatibility with the deployment framework.

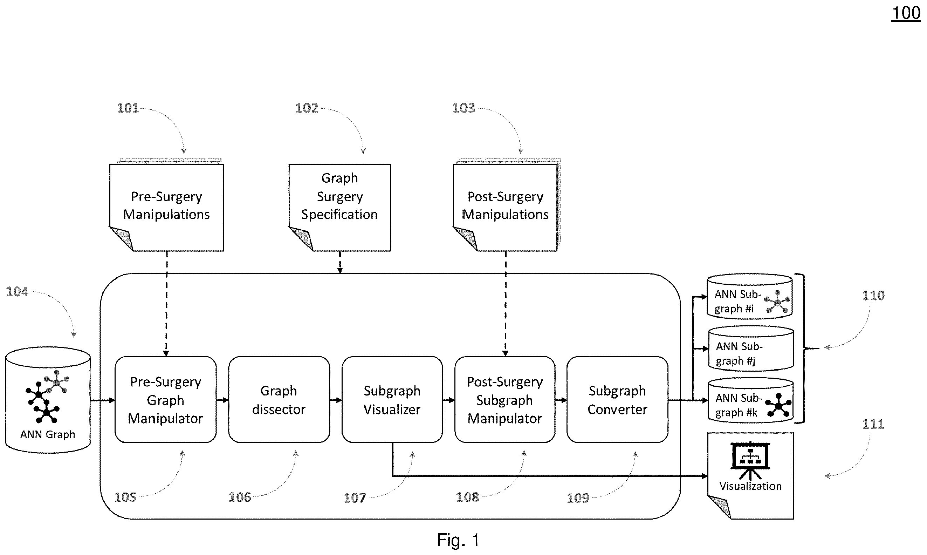

Fig. 1 shows an illustration 100 of a high-level block diagram of a system according to various embodiments. The inputs to the system may include pre-surgery manipulations 101, graph surgery specification 102, post-surgery manipulations 103, and an ANN graph 104. The outputs of the system may include a plurality of sub-graphs 110 and multiple forms of visualization 111, which may collectively constitute the interface of the invention toward the user. The main functional elements of the tool are indicated by blocks 105, 106, 107, 108, 109, which will be described in more detail below.

The input ANN 104, which may be the ML graph to be sliced, may be provided in an aforementioned static graph exchange format to the system, alongside a textual graph surgery specification file 102. An exemplary embodiment of the tool may start with converting the static input graph into an artificial neural network graph data structure which allows for easier programmatic manipulations. Next, pre-surgery manipulations, which may also be referred to as pre-surgery custom manipulations, may be executed by block 105, followed by the actual dissection of subgraphs in block 106 as determined in the graph surgery specification file. After dissection, the interface information of the carved-out subnetworks may become available for which graph surgery visualization takes place in block 107, outputting detailed depictions to the user as visualization 111. Next, post-surgery manipulations, which may also be referred to as post-surgery custom manipulations, may be executed by block 108. The post-surgery custom manipulations may be specific to the extracted subnetworks. Finally, each carved out subnetwork may be transformed via the subgraph converter module 109 into a desired output format which may also be provided in the graph surgery specification file, resulting in the carved out and converted subnetwork graphs 110, which may be provided in an onboard format.

According to various embodiments, as illustrated in Fig. 1, different categories of graph manipulations are distinguished:
Pre-surgery manipulations 101 and
Post-surgery manipulations 103.

This separation may provide that global graph-level manipulations, i.e. pre-surgery manipulations, and local subnetwork-level manipulations, i.e. post-surgery manipulations, may be applied independently from each other. Hence, also the separation of the pre-surgery graph manipulator 105 and the post-surgery graph manipulator 108, which may offer the same functionality except on different scopes, may be provided.

The graph surgery specification file 102 may be a human-readable textual file which can be given as a serializable data interchange format that uses attribute-value pairs. Herein, first user-defined pre-surgery graph manipulations may be enumerated which may be executed on the complete graph. This option may allow the user to plug in global pre-surgery manipulations 101 without disclosure, for which a software interface is specified. In other words, the user (for example customer, which may be a third-party) can create arbitrary manipulations (or optimizations) which they can invoke plug-and-play using the methods and systems according to various embodiments. Thus, the user does not need to provide details on the manipulation to be integrated into the methods and systems for usage. The user may simply use the methods and systems according to various embodiments as an external module, and thus, the manipulations remain confidential and undisclosed.

On the one hand, the interface as described herein may specify the input and output arguments that such custom manipulation need to follow to be applicable to various embodiments. On the other hand, the software interface may provide a set of utility functions which may be used in the custom code.

Next, the subnetworks may be specified by their designated names, as well as a list of input and output tensor names which delimit them. Furthermore, for each subnetwork, a list of user-defined post-surgery manipulations 103 may be provided as well. Herein, custom graph manipulations may be specified which are to be applied locally on individual subnetworks only. Lastly, a list of output formats may be specified for each subnetwork, which internally instructs the subgraph converter module 109 for the desired graph format specification of each subnetwork, e.g. an onboard format.

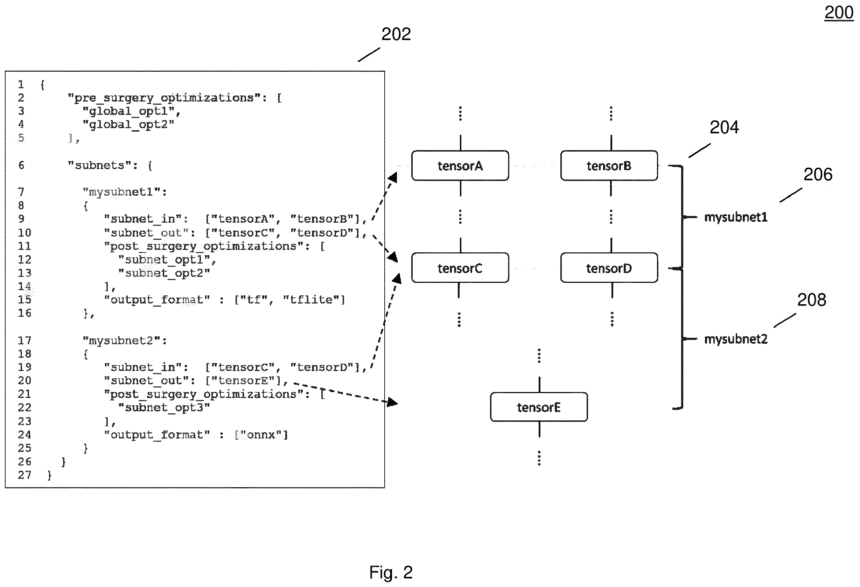

Fig. 2 shows an exemplary illustration 200 of a meta-implementation of a graph surgery specification file 202 and the corresponding subnetwork outputs 204 according to various embodiments.

In this basic example, the user-defined pre-surgery graph manipulations are given on Lines 2-5. In the depicted implementation two such exemplary custom manipulation passes are specified (Lines 3-4): global_opt1 and global_opt2. Starting on Line 6, the subnetworks are specified by their names. In this example, two connected subnets are specified as a desired dissection output: mysubnet1 (Line 7) and mysubnet2 (Line 17). Both of these are separated by a list of input tensors (Lines 9 and 19) and output tensors (Lines 10 and 20) which uniquely specify the interface of each subnetwork. The desired cutting points and the resulting dissection are highlighted on the right-hand side 204 of Fig. 2. For each of these subnets, exemplary post-surgery manipulations are specified as well. For mysubnet1 206 subnet_opt1 and subnet_opt2 (Lines 11-14), as well as for mysubnet2 208 subnet_opt3 (Lines 21-23). Lastly, the desired output format(s) of these subnetworks are specified on Lines 15 and 24 using names to enumerate specific ML graph exchange formats. In this example, the Tensorflow and TensorflowLite graph formats (see for example www.tensorflow.org) are specified for the conversion of mysubnet1 (Line 15) and ONNX (Open Neural Network Exchange, see for example onnx.ai) for mysubnet2 (Line 24).

As described above, pre-surgery manipulations carried out by the pre-surgery graph manipulator 105 are intended to be applied globally on the whole ML graph. Such global alterations may improve deployability and/ or execution performance of certain commonly occurring problematic patterns in the ML graph, but may also preserve the original functionality, leaving the model fully executable and functionally equivalent. The pre-surgery graph manipulator 105 may be invoked to execute such manipulation passes via a defined software interface and instructions given in the graph surgery specification file 102.

Various embodiments may use built-in generic graph manipulations after the user-provided pre-surgery manipulations. Such features may be provided by the software interfaces of standard exchange formats. Generic manipulations may include removing disconnected subgraphs and unused nodes, merging duplicated or fusible operations, folding constants, and many others. The main purpose of such generic manipulations is to clean up residual nodes arising as possible side-effects of the user-provided custom passes. This may ensure that unwanted artifacts do not get propagated to the stage of graph surgery.

An example of a pre-surgery manipulation may be folding so-called batch normalization layers into the predecessor convolutional or fully connected layer. Convolutional layers and fully connected layers are two types of layers commonly used in artificial neural networks and as such are well known in the art. Batch normalization may be essential during training and may significantly improve ANN performance. Yet, it adds an extra multiplication and addition per convolutional or fully connected layer in inference, which may provide significant overhead. Certain deployment tools cannot perform such folding automatically, but the custom manipulation pass may do.

After the initial graph manipulations, the graph dissector 106 may perform the actual cutting of the graph (in other words: graph dissection). For the concrete requirement of the dissection, the graph surgery specification 102 may be used. The graph dissector 106 may iterate over the desired subnetworks, may consolidate their interfaces, may check the validity of the graphs and may perform the dissection.

Next in the flow, the subgraph visualizer 107 may be invoked. The subgraph visualizer 107 may be a module that provides multiple forms of optical feedback to the user as outputs 111. This functionality may be integrated right after graph dissection since subnetwork interfaces have already been established at this point in the flow, for which providing extra visualization output is rather straightforward. According to various embodiments, the following depictions may be provided as outputs:
- A high-level representation of the graph surgery with subgraphs as single nodes and tensors, where the incisions took place, as edges. This visualization may provide a good interface-level overview on the performed graph surgery.
- A detailed graph surgery depiction with nodes as the mathematical operations within the ANN and tensors between them as edges. This depiction may represent nodes belonging to different subnetworks using different colors. This visualization may provide a supreme in-depth overview on graph surgery itself, the resulting subnetworks and their interconnections.

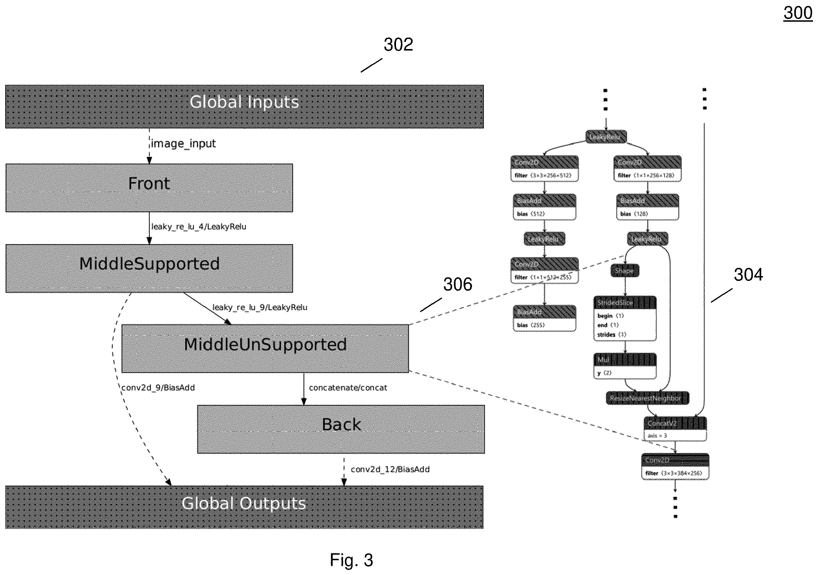

Fig. 3 shows an illustration of a high-level graph surgery visualization 302 (in the left portion of Fig. 3) and a detailed graph surgery visualization 304 (in the right portion of Fig. 3) according to various embodiments. In this practical example, graph surgery was performed on a state-of-the-art object detection and classification network called YOLOv3 (J. Redmon, A. Farhadi: "YOLOv3: An Incremental Improvement", arXiv:1804.02767). For the sake of the example, it may be assumed that certain mathematical operators in this network, specifically Shape, StridedSlice and ConvatV2, are not supported by the target deployment tool. Thus, the interfaces for graph surgery may be specified to group those parts of the network into a single subgraph where these operators appear.

The high-level visualization 302 of the performed surgery is shown on the left-hand side of Fig. 3. Herein, subgraphs are represented as single blocks (e.g., Front, MiddleSupported, MiddleUnSupported, Back) and the tensors that connect them appear as edges.

On the right side of Fig. 3, an excerpt of the detailed graph surgery visualization is shown using open-source visualization tool Netron (see for example https://netron.app/). In this representation, all mathematical operations in subnetworks appear as nodes including many details. The tensors that connect these operators appear as arrows between them. The graph surgery visualization may color code all nodes to distinguish the subnetworks in which they reside. Herein, operations that are assumed unsupported in the example were grouped in the MiddleUnSupported subnetwork 306. In a practical scenario, this subgraph would need to be either manually re-implemented or deployed using possibly another deployment tool (if available), that supports the aforementioned operations.

In the following, post-surgery manipulations according to various embodiments will be described.

The post-surgery manipulator 108 may be similar to the pre-surgery graph manipulator 105, but this time on the subgraph level: As stated before, post-surgery manipulations 103 may be intended to be applied individually on carved out subnetworks. Again, the goal may be to improve deployability and/ or execution performance of commonly occurring problematic patterns in subgraphs. Yet, the alterations herein may break the original functionality as well as functional equivalence in favor of deployability, hence the local scope.

The post-surgery subgraph manipulator 108 may follow the same software interface and may provide the same utility functions as its pre-surgery counterpart. Yet, the post-surgery subgraph manipulator 108 may apply user-specified post-surgery manipulations 103 locally on individual subnets. Similar to the pre-surgery manipulation, built-in generic graph manipulation may follow the post-surgery manipulation stage, for example to clean up any residual nodes.

An example of a post-surgery manipulations may be tiling, in other words: converting, large convolutions into smaller ones. This may be achieved by either reducing the height and width of their feature maps or by splitting the convolutions by their channel dimension. Feature maps may be understood as an image like data structure that includes values (in other words: features) that are output or intermediate results of an artificial neural network. On the one hand, such a manipulation may aim at fitting convolutions, e.g., to the panel size of the target hardware accelerator for an efficient utilization. On the other hand, convolution splitting may aim at reducing the tensors of convolutions so that they fit on the on-chip memory of the device rather than falling back to the off-chip memory. The on-chip memory is faster than the off-chip memory. Since such a change may be hardware-specific, it may be implemented as a post-surgery manipulation.

In the following, a subgraph converter according to various embodiments will be described.

Lastly, the dissected subnets may be transformed by the subgraph converter 109 from their artificial neural network graph data structures to the on-target format(s) 110. To govern this process, the graph surgery specification file 102 may be used, defining all required settings for the transformation. Such requirements may include, e.g., specifying the model exchange format, and the data type of conversion, among others. As for the latter option, various embodiments may enable to transform the subnet from its original floating-point to a more light-weight integer numerical representation, a technique which is referred to as quantization. This may allow compressing the network and reducing computational and memory requirements, making the final output more embedded-friendly. Quantization, however, may require certain input statistics to the network, for which data can be provided using also the graph surgery specification.

In the following, various effects and details of various embodiments will be described.

Various embodiments may provide flexible graph-level surgery and manipulation of artificial neural networks.

Various embodiments may provide a semantic separation between the training and deployment flows.

Various embodiments may use a simple textual configuration file instead of software interfaces to perform graph surgery and to select the specific manipulations to be applied. This may enable the most essential steps of graph surgery to be performed simply and tool-agnostically. Moreover, such files may allow to snapshot different cut and manipulation configurations for a graph, which may be used for debugging, as well as tracking the progress of deployment.

Various embodiments may provide a software interface for the user to integrate custom pre-surgery graph manipulations on the global graph, as well as post-surgery manipulations on particular subnetworks. Besides the mechanism for custom manipulations, a set of built-in graph-level manipulations may be provided, which are generally applied on the full graph.

Various embodiments may separate the concept of pre- and post-surgery manipulations, on top of generic graph manipulations, which notions may be imminent in practical graph surgery flows.

Various embodiments may provide multiple forms of intuitive visual feedback to the user to inspect, understand and verify the results of graph surgery.

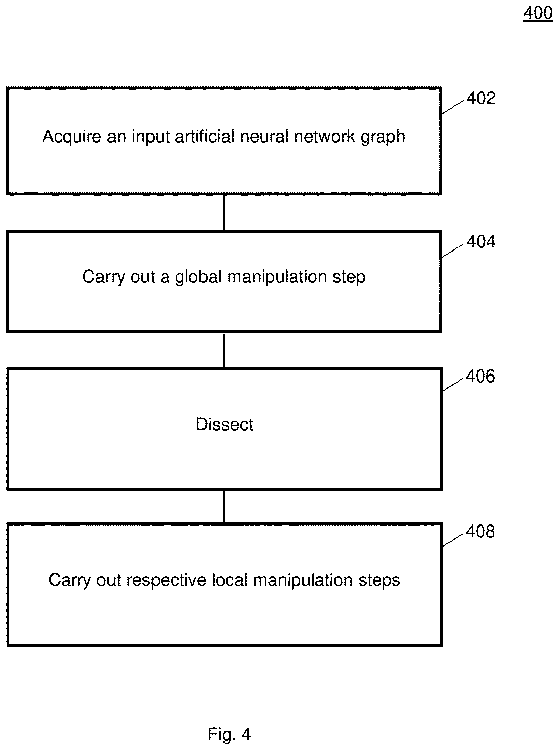

Fig. 4 shows a flow diagram 400 illustrating a method for adapting an artificial neural network graph according to various embodiments. At 402, an input artificial neural network graph may be acquired. At 404, a global manipulation step comprising a first set of manipulations may be carried out. The first set of manipulation may be configured to adapt the input artificial neural network graph based on at least one user-defined criterion and generate a first intermediate artificial neural network graph. At 406, the first intermediate artificial neural network graph may be dissected to generate a plurality of second intermediate artificial neural network graphs based on a specification file. At 408, a respective local manipulation step may be carried out for each of the plurality of second intermediate artificial neural network graphs, wherein each local manipulation step may include a corresponding second set of manipulations configured to adapt a corresponding second intermediate artificial neural network graph based on at least one corresponding user-defined criterion and generate a corresponding manipulated second intermediate artificial neural network graph.

According to various embodiments, the at least one manipulated second intermediate artificial neural network graph may be converted to an on-board format that is directly executable on the target hardware / embedded system

According to various embodiments, the first set of manipulations and/or the second set of manipulations may be provided in a configuration file, preferably a textual configuration file.

According to various embodiments, dissecting may include or may be dissecting the first intermediate artificial neural network graph at pre-defined points of intersection.

According to various embodiments, the points of intersection may be provided in a configuration file, preferably a textual configuration file.

According to various embodiments, the input artificial neural network graph may be provided in an off-board format.

According to various embodiments, carrying out the respective local manipulation step may include or may be carrying out individual manipulations to at least two second intermediate artificial neural network graphs.

According to various embodiments, each of the input artificial neural network graph, the first intermediate artificial neural network graph, the plurality of second intermediate artificial neural network graphs, and the at least one manipulated second intermediate artificial neural network graph may include a respective plurality of nodes representing mathematical operations and a respective plurality of edges representing tensors.

According to various embodiments, the method may further include visualizing at least one graph selected from a list of graphs consisting of: the input artificial neural network graph, the first intermediate artificial neural network graph, the plurality of second intermediate artificial neural network graphs, and the at least one manipulated second intermediate artificial neural network graph.

According to various embodiments, the at least one manipulated second intermediate artificial neural network graph may be to be deployed on a resource-constrained embedded system.

According to various embodiments, the embedded system may be a mobile computing device, a mobile phone, a tablet computing device, an automotive compute platform, or an edge device.

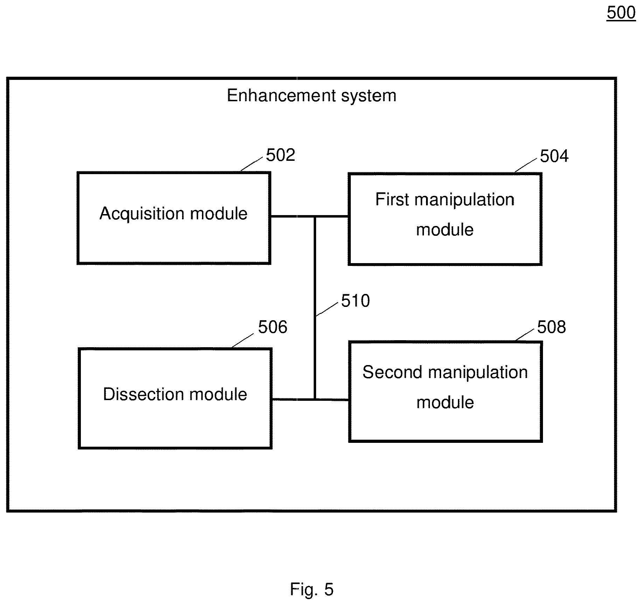

Fig. 5 shows an enhancement system 500 according to various embodiments. The enhancement system 500 may include an acquisition module 502, a first manipulation module 504, a dissection module 506, and a second manipulation module 508.
The acquisition module 502 may be configured to acquire an input artificial neural network graph;
The first manipulation module 504 may be configured to carry out a global manipulation step including a first set of manipulations configured to adapt the input artificial neural network graph based on at least one user-defined criterion and generate a first intermediate artificial neural network graph;
The dissection module 506 may be configured to dissect the first intermediate artificial neural network graph to generate a plurality of second intermediate artificial neural network graphs based on a specification file.

The second manipulation module 508 may be configured to carry out a respective local manipulation step for each of the plurality of second intermediate artificial neural network graphs, wherein each local manipulation step includes a corresponding second set of manipulations configured to adapt a corresponding second intermediate artificial neural network graph based on at least one corresponding user-defined criterion and generate a corresponding manipulated second intermediate artificial neural network graph.

The acquisition module 502, the first manipulation module 504, the dissection module 506, and the second manipulation module 508 may be coupled with each other, e.g. via an electrical connection 510, such as e.g. a cable or a computer bus or via any other suitable electrical connection to exchange electrical signals.

A "module" may be understood as any kind of a logic implementing entity, which may be special purpose circuitry or a processor executing a program stored in a memory, firmware, or any combination thereof.

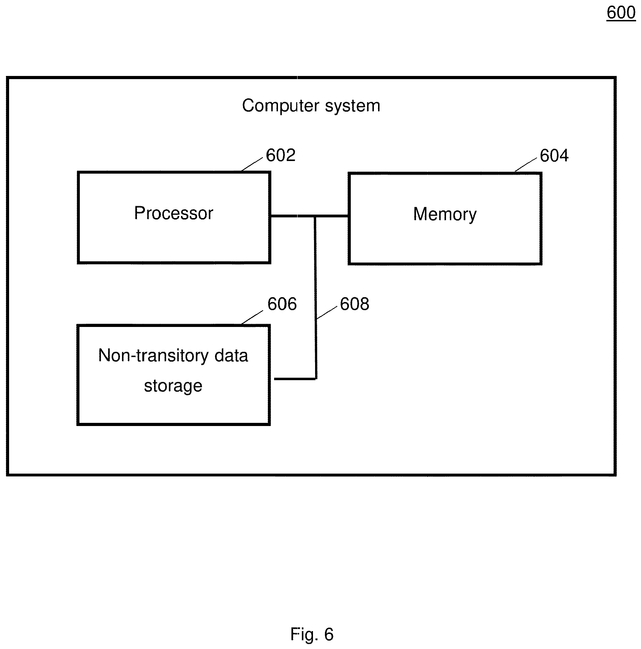

Fig. 6 shows a computer system 600 with a plurality of computer hardware components configured to carry out steps of a computer implemented method for adapting an artificial neural network graph according to various embodiments. The computer system 600 may include a processor 602, a memory 604, and a non-transitory data storage 606.

The processor 602 may carry out instructions provided in the memory 604. The non-transitory data storage 606 may store a computer program, including the instructions that may be transferred to the memory 604 and then executed by the processor 602.

The processor 602, the memory 604, and the non-transitory data storage 606 may be coupled with each other, e.g. via an electrical connection 608, such as e.g. a cable or a computer bus or via any other suitable electrical connection to exchange electrical signals.

The terms "coupling" or "connection" are intended to include a direct "coupling" (for example via a physical link) or direct "connection" as well as an indirect "coupling" or indirect "connection" (for example via a logical link), respectively.

It will be understood that what has been described for one of the methods above may analogously hold true for the enhancement system 500 and/or for the computer system 600.

### Reference numeral list

- 100: illustration of a high-level block diagram of a system according to various embodiments

- 101: pre-surgery manipulations
- 102: graph surgery specification
- 103: post-surgery manipulations
- 104: ANN graph
- 105: pre-surgery graph manipulator
- 106: graph dissector
- 107: subgraph visualizer
- 108: post-surgery subgraph manipulator
- 109: subgraph converter
- 110: plurality of sub-graphs
- 111: visualization

- 200: illustration of a meta-implementation of a graph surgery specification file and the corresponding subnetwork outputs according to various embodiments

- 202: meta-implementation of a graph surgery specification file
- 204: corresponding subnetwork outputs
- 206: first subnet
- 208: second subnet

- 300: illustration of a high-level graph surgery visualization and a detailed graph surgery visualization according to various embodiments
- 302: high-level graph surgery visualization
- 304: detailed graph surgery visualization
- 306: MiddleUnSupported subnetwork
- 400: flow diagram illustrating a method for adapting an artificial neural network graph according to various embodiments
- 402: step of acquiring an input artificial neural network graph
- 404: step of carrying out a global manipulation step
- 406: step of dissecting
- 408: step of carrying out respective local manipulation steps

- 500: enhancement system
- 502: acquisition module
- 504: first manipulation module
- 506: dissection module
- 508: second manipulation module
- 510: connection

- 600: computer system according to various embodiments
- 602: processor
- 604: memory
- 606: non-transitory data storage
- 608: connection

## Claims

1. Computer implemented method for adapting an artificial neural network graph, the method comprising:
- acquiring (402) an input artificial neural network graph;
- carrying out (404) a global manipulation step comprising a first set of manipulations configured to adapt the input artificial neural network graph based on at least one user-defined criterion and generate a first intermediate artificial neural network graph;
- dissecting (406) the first intermediate artificial neural network graph to generate a plurality of second intermediate artificial neural network graphs based on a specification file (202); and
- carrying out (408) a respective local manipulation step for each of the plurality of second intermediate artificial neural network graphs, wherein each local manipulation step comprises a corresponding second set of manipulations configured to adapt a corresponding second intermediate artificial neural network graph based on at least one corresponding user-defined criterion and generate a corresponding manipulated second intermediate artificial neural network graph.

2. The computer implemented method of claim 1, further comprising:
- converting the at least one manipulated second intermediate artificial neural network graph to an on-board format that is directly executable on the target hardware / embedded system

3. The computer implemented method of at least one of claims 1 or 2, wherein the first set of manipulations and/or the second set of manipulations are provided in a configuration file, preferably a textual configuration file.

4. The computer implemented method of at least one of claims 1 to 3, wherein dissecting comprises dissecting the first intermediate artificial neural network graph at pre-defined points of intersection.

5. The computer implemented method of claim 4,
wherein the points of intersection are provided in a configuration file, preferably a textual configuration file.

6. The computer implemented method of at least one of claims 1 to 5, wherein the input artificial neural network graph is provided in an off-board format.

7. The computer implemented method of at least one of claims 1 to 6, wherein carrying out the respective local manipulation step comprises carrying out individual manipulations to at least two second intermediate artificial neural network graphs.

8. The computer implemented method of at least one of claims 1 to 7, wherein each of the input artificial neural network graph, the first intermediate artificial neural network graph, the plurality of second intermediate artificial neural network graphs, and the at least one manipulated second intermediate artificial neural network graph comprises a respective plurality of nodes representing mathematical operations and a respective plurality of edges representing tensors.

9. The computer implemented method of at least one of claims 1 to 8, further comprising:
visualizing at least one graph selected from a list of graphs consisting of:
the input artificial neural network graph, the first intermediate artificial neural network graph, the plurality of second intermediate artificial neural network graphs, and the at least one manipulated second intermediate artificial neural network graph.

10. The computer implemented method of at least one of claims 1 to 9, wherein the at least one manipulated second intermediate artificial neural network graph is to be deployed on a resource-constrained embedded system.

11. The computer implemented method of claim 10,
wherein the embedded system is a mobile computing device, a mobile phone, a tablet computing device, an automotive compute platform, or an edge device.

12. Computer system, the computer system comprising a plurality of computer hardware components configured to carry out steps of the computer implemented method of at least one of claims 1 to 11.

13. Non-transitory computer readable medium comprising instructions for carrying out the computer implemented method of at least one of claims 1 to 11.
